# EUROPÄISCHE PATENTSCHRIFT

(11) **EP 0 975 719 B1**
(45) Veröffentlichungstag und Bekanntmachung des Hinweises auf die Patenterteilung: **11.12.2002**
(21) Anmeldenummer: 98924086.6
(22) Anmeldetag: 27.03.1998
(51) Int. Cl.: C11D 1/29, C11D 1/16, C11D 17/00

(54) **VERFAHREN ZUR HERSTELLUNG VON NIEDRIGVISKOSEN WÄSSERIGEN ALKYL(ETHER)SULFATPASTEN**
PROCESS FOR PREPARING LOW VISCOSITY, HIGHLY CONCENTRATED, AQUEOUS ALKYL(ETHER) SULFATE PASTES
PROCEDE DE PREPARATION DES PATES AQUEUSES D'ALKYL(ETHER)SULFATE A BASSE VISCOSITE ET A HAUTE CONCENTRATION

(30) Priorität: 05.04.1997 DE 19714043
(43) Veröffentlichungstag der Anmeldung: 02.02.2000
(73) Patentinhaber: Cognis Deutschland GmbH & Co. KG, 40589 Düsseldorf (DE)
(72) Erfinder: LE HEN FERRENBACH, Catherine, F-77100 Meaux (FR); GUEGAN, Michel, F-77165 Iverny (FR)
(86) Internationale Anmeldenummer: EP9801821
(87) Internationale Veröffentlichungsnummer: WO98045391

(56) Entgegenhaltungen:
- DE-A- 2 326 006
- DE-A- 2 935 428
- DE-A- 4 032 910
- DE-A- 4 438 583
- DE-A- 4 446 444
- US-A- 4 765 926
- J. FALBE: "SURFACTANTS IN CONSUMER PRODUCTS", 1987, SPRINGER-VERLAG, BERLIN

## Beschreibung

### Gebiet der Erfindung

Die Erfindung betrifft ein Verfahren zur Herstellung von niedrigviskosen, hochkonzentrierten Alkyl(ether)sulfatpasten durch Cosulfatierung von Alkylpolyglycolethern und Glycerinen.

### Stand der Technik

Alkylsulfate und -ethersulfate stellen bekannte anionische Tenside dar, die man großtechnisch durch Sulfatierung von Alkoholen bzw. Alkylpolyglycolethern mit Schwefeltrioxid und nachfolgende Neutralisation erhält. Wegen ihres aus-gezeichneten Schaumvermögens, der hohen Schaumstabilität, Härteund Fettbelastbarkeit sowie der guten hautkosmetischen Verträglichkeit stellen Alkyl(ether)sulfate Basistenside für die Herstellung vor allem von Haarshampoos und manuellen Geschirrspülmitteln dar. Es ist sofort klar, daß für den Hersteller von Alkyl(ether)sulfaten ein starkes Bedürfnis besteht, diese Tenside in Form möglichst hoch konzentrierter wäßriger Zubereitungen zur Verfügung zu stellen, um überflüssige Transport- und Lagerkosten zu vermeiden. Gleichzeitig ist aber bekannt, daß anionische Tenside im allgemeinen und Alkyl(ether)sulfate im besonderen bei höheren Feststoffgehalten vergelen bzw. hochviskose Phasen bilden, die das Umfüllen und Pumpen erschweren.

In diesem Zusammenhang hat es natürlich nicht an Versuchen gemangelt, das Problem des unzureichenden rheologischen Verhaltens zu verbessern. Insbesondere der Zusatz viskositätsmindernder Stoffe, wie beispielsweise sulfatierter Polyethylenglycole [**DE-AS 2935428** (Henkel)] oder sulfierter Triglyceride wurde diskutiert. In der Praxis erweisen sich diese Stoffe für die Viskositätsregulierung gerade von Alkylethersulfaten aber als nicht sehr geeignet. Aus der **DE-A1 4446362** (Henkel) ist ein Verfahren zur Herstellung von konzentrierten Pasten von Alkylethersulfat-Magnesiumsalzen bekannt, bei dem man die aus der Sulfatierung resultierenden rohen Sulfatierungsprodukte kontinuierlich in einen Neutralisationskreislauf eindosiert, der zu Beginn eine konzentrierte wäßrige Fettalkoholethersulfat-Natriumsalz-Paste als Vorlage enthält, das resultierende Vorgemisch mit einer wäßrigen Erdalkalihydroxid/Alka-nolamin-Dispersion neutralisiert, und die resultierende neutralisierte konzentrierte Erdalkalisalz-Paste in den Neutralisationskreislauf zurückführt, bis das Endprodukt praktisch frei von Natriumsalzen ist. Das Verfahren eignet sich aber nur für die Herstellung von Magnesiumsalzen und ist mit hohem tech-nischen Aufwand verbunden.

Aus der deutschen Offenlegungsschrift **DE-A1 4446444** (Henkel) ist ein Verfahren zur Herstellung von wasserfreien Tensiden bekannt, bei dem man wäßrige Aufschlämmungen von anionischen und/oder nichtionischen Tensiden in Gegenwart von Glycerinsulfaten einer Sprühtrocknung unterwirft. Gegenstand der deutschen Offenlegungsschrift **DE-A1 4446371** (Henkel) ist ein Verfahren zur Herstellung von Glycerinsulfaten, bei dem man zur Sulfatierung von Glycerin ein SO₃/Inertgasgemisch mit einem SO₃-Anteil von 1 bis 4,5 Vol-% einsetzt. Schließlich wird in der deutschen Offenlegungsschrift **DE-A1 4438583** (Henkel) der Einsatz von Glycerinsulfaten als Lösungsvermittler in hochkonzentrierten Handgeschirrspülmitteln vorgeschlagen.

Demzufolge hat die Aufgabe der Erfindung darin bestanden, ein Verfahren zur Verfügung zu stellen, mit dessen Hilfe man ohne großen technischen Aufwand wäßrige Alkylethersulfatpasten erhält, die auch bei hohem Feststoffgehalt noch eine ausreichend niedrige Viskosität aufweisen.

### Beschreibung der Erfindung

Glycerinsulfate der Formel **(I)**, 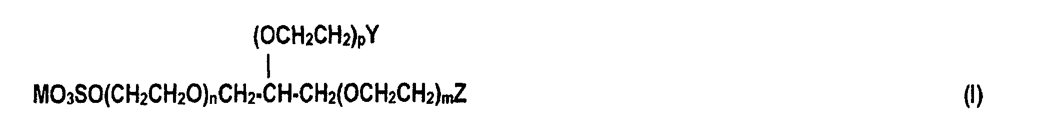 in der Y und Z unabhängig voneinander für eine Hydroxylgruppe oder eine SO₃M-Gruppe, M für Wasserstoff, ein Alkali- oder Erdalkalimetall und die Summe (n+m+p) für 0 oder Zahlen von 1 bis 50 steht, werden durch das erfindungsgemäße Verfahren erhalten.

### Glycerinsulfate

Glycerinsulfate stellen bekannte Stoffe dar, die man nach den einschlägigen Methoden der präparativen organischen Chemie herzustellen vermag. Typischerweise geht man von Glycerin oder einem statistischen Anlagerungsprodukt von 1 bis 50, vorzugsweise 2 bis 25 und insbesondere 5 bis 15 Mol Ethylenoxid an Glycerin aus, die bei Temperaturen im Bereich von 15 bis 50°C mit einer solchen Menge gasförmigem Schwefeltrioxid oder Chlorsulfonsäure in Kontakt gebracht werden, daß eine, zwei oder drei Hydroxylgruppen sulfatiert werden. Demzufolge kann das molare Einsatzverhältnis Glycerin bzw. Glycerin+EO-Addukt zu Sulfatierungsmittel im Bereich 1 : 0,25 bis 1 : 1,1 liegen. Vorzugsweise wählt man das molare Verhältnis so, daß im Mittel zwei Hydroxylgruppen sulfatiert werden. Dabei kann es im übrigen auch zur Ausbildung von cyclischen Sulfaten mit 5-Ring- und 6-Ring-Struktur kommen. Die Neutralisation - falls erwünscht - findet in an sich bekannter Weise statt, d.h. entweder trägt man die sauren Sulfate in eine entsprechende wäßrige Neutralisationsbase, vorzugsweise 25 bis 55 Gew.-%ige Natronlauge ein, oder man führt eine kontinuierliche Neutralisation durch, indem man Ströme des sauren Sulfierproduktes und der wäßrigen Base miteinander vermischt.

Üblicherweise setzt man die Glycerinsulfate in Form ihrer Salze und dann vorzugsweise als wäßrige, etwa 10 bis 50 Gew.-%ige Lösungen ein. Andererseits ist aber auch der Einsatz der nichtneutralisierten sauren Sulfierprodukte möglich. Dies hat den Vorteil, daß kein weiteres Wasser in das Produkt eingetragen wird, da die sauren Glycerinsulfate auch wasserfrei flüssig und niedrigviskos sind und daher leicht eindosiert werden können. Da Aniontenside mit Sulfatstruktur jedoch im sauren Bereich nicht beständig sind, ist in diesem Fall darauf zu achten, daß die Alkylethersulfatpasten alkalisch eingestellt sind und die Alkalireserve ausreicht, um die sauren Glycerinsulfate in der Paste zu neutralisieren.

### Alkyl(ether)sulfate

Alkylsulfate und Alkylethersulfate ("Ethersulfate") stellen bekannte anionische Tenside dar, die großtechnisch durch SO₃- oder Chlorsulfonsäure (CSA)-Sulfatierung von Fettalkoholen oder Oxoalkoholen bzw. Fettalkohol- oder Oxoalkoholpolyglycolethern und nachfolgende Neutralisation hergestellt werden. Im Sinne der Erfindung kommen Alkylsulfate und -ethersulfate in Betracht, die der Formel (II) folgen,

**R**^{**1**}**O-(CH**_{**2**}**CH**_{**2**}**O)**_{**q**}**SO**_{**3**}**X (II)**

in der R¹ für einen linearen oder verzweigten Alkyl- und/oder Alkenylrest mit 6 bis 22 Kohlenstoffatomen, q für 0 oder Zahlen von 1 bis 10 und X für ein Alkali- und/oder Erdalkalimetall, Ammonium, Alkylammonium, Alkanolammonium oder Glucammonium steht. Typische Beispiele sind die Sulfate von Anlagerungsprodukten von durchschnittlich 1 bis 10 und insbesondere 2 bis 5 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen, in Form ihrer Natrium- und/oder Magnesiumsalze. Die Ethersulfate können dabei sowohl eine konventionelle als auch eine eingeengte Homologenverteilung aufweisen. Besonders bevorzugt ist der Einsatz von Kokosalkylsulfaten oder Ethersulfaten auf Basis von Addukten von durchschnittlich 2 bis 3 Mol Ethylenoxid an technische C_{12/14}- bzw. C_{12/18}-Kokosfettalkoholfraktionen in Form ihrer Natrium- und/oder Magnesiumsalze. Üblicherweise werden wäßrige Pasten von Alkyl(ether)sulfaten eingesetzt, die einen Feststoffgehalt im Bereich von 50 bis 80, vorzugsweise 60 bis 70 Gew.-% aufweisen. Die Zusatzmenge der Glycerinsulfate beträgt in der Regel 0,1 bis 5, vorzugsweise 1 bis 3 Gew.-% - bezogen auf den Feststoffanteil der Pasten.

### Cosulfatierung

In einer bevorzugten Ausführungsform der Erfindung werden die Glycerinsulfate nicht separat hergestellt und dann den Alkyl(ether)sulfatpasten zugesetzt, sondern durch eine Cosulfatierung in situ erzeugt. Ein weiterer Gegenstand der Erfindung betrifft daher ein Verfahren zur Herstellung von niedrigviskosen, konzentrierten wäßrigen Alkyl(ether)sulfatpasten, bei dem man Mischungen von
(a) primären Alkoholen bzw. Alkylpolyglycolethern der Formel (III),

   **R**^{**1**}**O-(CH**_{**2**}**CH**_{**2**}**O)**_{**q**}**H (III)**

   in der R¹ für einen linearen oder verzweigten Alkyl- und/oder Alkenylrest mit 6 bis 22 Kohlenstoffatomen und q für 0 oder Zahlen von 1 bis 10 steht, und
(b) Glycerin und/oder Anlagerungsprodukte von durchschnittlich 1 bis 50 Mol Ethylenoxid an Glycerin
gemeinsam sulfatiert und anschließend mit einer solchen Menge einer wäßrigen Neutralisationsbase versetzt, daß sich ein pH-Wert von 7,5 bis 9 und eine Feststoffkonzentration von 50 bis 80 einstellt. Üblicherweise setzt man die Komponenten (a) und (b) im Gewichtsverhältnis 95 : 5 bis 99,9 : 0,1 und vorzugsweise 97 : 3 bis 99 : 1 ein.

Die Umsetzung der Ausgangsstoffe mit gasförmigem Schwefeltrioxid kann in der für Fettsäureniedrigalkylester bekannten Weise **[J.Falbe (ed.), "Surfactants in consumer products"; Springer Verlag, Berlin-Heidelberg, 1987, S.61]** erfolgen, wobei Reaktoren, die nach dem Fallfilmprinzip arbeiten, bevorzugt sind. Dabei wird das Schwefeltrioxid mit einem inerten Gas, vorzugsweise Luft oder Stickstoff verdünnt und in Form eines Gasgemisches, welches das Sulfieragens in einer Konzentration von 1 bis 8, insbesondere 2 bis 5 Vol.-% enthält, eingesetzt. Das molare Einsatzverhältnis von Ausgangsstoff zu Schwefeltrioxid beträgt 1 : 0,95 bis 1 : 1,8, vorzugsweise jedoch 1 : 1,0 bis 1 : 1,6. und insbesondere 1 : 1,3 bis 1 : 1,5. Die Sulfierreaktion wird bei Temperaturen von 35 bis 90, vorzugsweise 35 bis 80°C durchgeführt.

Die bei der Reaktion anfallenden sauren Sulfierprodukte werden in wäßrige Basen eingerührt, neutralisiert und auf einen pH-Wert von 6,5 bis 8,5 eingestellt. Die Neutralisation wird mit Basen ausgewählt aus der von Alkalimetallhydroxiden wie Natrium-, Kalium- und Lithiumhydroxid, Erdalkalimetalloxiden und -hydroxiden wie Magnesiumoxid, Magnesiumhydroxid, Calciumoxid und Calciumhydroxid, Ammoniak, Mono-, Di- und Tri-C₂₋₄-Alkanolaminen, beispielsweise Mono-, Di- und Triethanolamin sowie primären, sekundären oder tertiären C₁₋₄-Alkylaminen gebildeten Gruppe durchgeführt. Die Neutralisationsbasen gelangen dabei vorzugsweise in Form 5 bis 55 Gew.-%iger wäßriger Lösungen zum Einsatz, wobei 25 bis 50 Gew.-%ige wäßrige Natriumhydroxidlösung bevorzugt ist.

Die Sulfatierungsprodukte können nach der Neutralisation in an sich bekannter Weise durch Zusatz von Wasserstoffperoxid- oder Natriumhypochloritlösung gebleicht werden, um eine für viele Anwendungen erwünschte weitere Farbaufhellung zu erreichen. Dabei werden, bezogen auf den Feststoffgehalt in der Lösung der Sulfierprodukte, 0,2 bis 2 Gew.-% Wasserstoffperoxid, berechnet als 100 Gew.-%ige Substanz, oder entsprechende Mengen Natriumhypochlorit eingesetzt. Der pH-Wert der Lösungen kann unter Verwendung geeigneter Puffermittel, z. B. mit Natriumphosphat oder Citronensäure konstant gehalten werden. Zur Stabilisierung gegen Bakterienbefall empfiehlt sich ferner eine Konservierung, z. B. mit Formaldehydlösung, p-Hydroxybenzoat, Sorbinsäure oder anderen bekannten Konservierungsstoffen.

### Gewerbliche Anwendbarkeit

Die erfindungsgemäß erhältlichen konzentrierten Alkylethersulfatpasten weisen eine niedrige Viskosität auf und eignen sich zur Herstellung beispielsweise von Handgeschirrspülmitteln oder Haarshampoos, in denen sie in Mengen von 1 bis 50, vorzugsweise 5 bis 25 Gew.-% - bezogen auf die Mittel - enthalten sein können.

### Beispiele

Einer wäßrigen Paste von Kokosfettalkohol+2EO-sulfat-Natriumsalz (Texapon N 70, pH= 8) mit einer Feststoffkonzentration von ca. 70 Gew.-% wurden 1 bis 5 Gew.-% verschiedener Viskositätsregulatoren zugesetzt und die Viskosität bei 20°C nach der Brookfield-Methode (RVT-Viskosimeter, Spindel 1, 10 UpM) bestimmt. Die Ergebnisse sind in Tabelle 1 zusammengefaßt. Die kursiv gedruckten Viskositäten entsprechen erfindungsgemäßen Beispielen, die übrigen dienen zum Vergleich.

**Tabelle 1**

| **Viskositätsmessungen; Angabe der Viskosität in [mPas], Angabe der Einsatzmenge bezogen auf Feststoffgehalt** | | | | | | |
|---|---|---|---|---|---|---|
| **Viskositätsregulatoren** | **Zusatzmenge* [Gew.-%]** | | | | | |
| | **0** | **1** | **2** | **3** | **4** | **5** |
| Glycerinmonosulfat, nicht neutralisiert | *4.000* | 3.900 | 3.700 | 3.500 | 3.200 | 3.000 |
| Glycerindisulfat, nicht neutralisiert | *4.000* | 3.900 | 3.700 | 3.400 | 3.300 | 3.000 |
| Glycerintrisulfat, nicht neutralisiert | *4.000* | 3.900 | 3.800 | 3.500 | 3.300 | 3.000 |
| Glycerinmonosulfat-Natriumsalz | *4.000* | 3.900 | 3.700 | 3.300 | 3.200 | 3.000 |
| Glycerindisulfat-Natriumsalz | *4.000* | 3.800 | 3.600 | 3.100 | 3.000 | 2.900 |
| Glycerindisulfat-Natriumsalz** | *4.000* | 3.700 | 3.500 | 3.000 | 2.900 | 2.800 |
| Glycerintrisulfat-Natriumsalz | *4.000* | 3.900 | 3.700 | 3.500 | 3.000 | 3.000 |
| Glycerin+10EO-sulfat-Natriumsalz | *4.000* | 3.800 | 3.700 | 3.500 | 3.100 | 2.700 |
| PEG-100-disulfat-Natriumsalz | *4.000* | *4.000* | *3.900* | *3.700* | *3.600* | *3.500* |
| PEG-1000-disulfat-Natriumsalz | *4.000* | *4.000* | *3.900* | *3.800* | *3.800* | *3.800* |

| | | | | | | |
|---|---|---|---|---|---|---|
| *) Glycerinsulfat-Salze wurden in Form 30 Gew.-%iger wäßriger Lösungen eingesetzt | | | | | | |
| **) Hergestellt durch Cosulfatierung | | | | | | |

## Patentansprüche

1. Verfahren zur Herstellung von niedrigviskosen, konzentrierten wässrigen Alkyl(ether)sulfatpasten, bei dem man Mischungen von
(a) primären Alkoholen bzw. Alkylpolyglycolethern der Formel (III),
**R**^{**1**}**O-(CH**_{**2**}**CH**_{**2**}**O)**_{**q**}**H (III)**
in der R¹ für einen linearen oder verzweigten Alkyl- undloder Alkenylrest mit 6 bis 22 Kohlenstoffatomen und q für 0 oder Zahlen von 1 bis 10 steht, und
(b) Glycerin und/oder Anlagerungsprodukte von durchschnittlich 1 bis 50 Mol Ethylenoxid an Glycerin
gemeinsam sulfatiert und anschließend mit einer solchen Menge einer wäßrigen neutralisations-base versetzt, dass sich ein pH-Wert von 7,5 bis 9 und eine Feststoffkonzentration von 50 bis 80 einstellt.

2. Verfahren nach Anspruch 6, **dadurch gekennzeichnet, dass** man die Komponenten (a) und (b) im Gewichtsverhältnis 95 : 5 bis 99,9 : 0,1 einsetzt.

## Claims

1. A process for the production of low-viscosity concentrated water-containing alkyl(ether) sulfate pastes in which mixtures of
(a) primary alcohols or alkyl polyglycol ethers corresponding to formula (III):
R¹O-(CH₂CH₂O)_{q}H (III)
in which R¹ is a linear or branched alkyl and/or alkenyl group containing 6 to 22 carbon atoms and q is 0 or a number of 1 to 10, and
(b) glycerol and/or products of the addition of on average 1 to 50 mol ethylene oxide onto glycerol
are sulfated together, after which an aqueous neutralization base is added in such a quantity that a pH value of 7.5 to 9 and a solids concentration of 50 to 80 are established.

2. A process as claimed in claim 6, **characterized in that** components (a) and (b) are used in a ratio by weight of 95:5 to 99.9:0.1.

## Revendications

1. Procédé de préparation de pâtes d'alkyl (éther) sulfates concentrées et de faible viscosité, dans lequel on sulfate ensemble des mélanges de :
a) des alcools primaires ou des éthers d'alkyl et de polyglycol de formule III
R¹O-(CH₂CH₂O)_{q}H (III)
dans laquelle R¹ représente un reste alkyle et/ou alkényle linéaire ou ramifié ayant de 6 à 22 atomes de carbone, et q représente O ou des nombres allant de 1 à 10,
et
b) du glycérol et/ou des produits d'addition de en moyenne 1 à 50 mol d'oxyde d'éthylène sur le glycérol,
et ensuite on mélange avec une quantité de base de neutralisation aqueuse telle qu'une valeur de pH de 7,5 à 9 et une concentration en matière solide de 20 à 80 - s'ajustent.

2. Procédé de préparation de pâtes selon la revendication 6,
**caractérisé en ce qu'**
on met en oeuvre les composants (a) et (b) dans un rapport en poids de 95 : 5 à 99,9 : 0,1.
